Europäisches Patentamt

European Patent Office

Office européen des brevets

(11) Publication number: **0 319 863**
**A2**

## EUROPEAN PATENT APPLICATION

(21) Application number: **88120196.6**

(22) Date of filing: **03.12.88**

(51) Int. Cl.⁴: **G01N 33/84 , //C07D323/00, C07C103/34,C07C103/737, C07C101/18**

(30) Priority: **11.12.87 US 131999**

(43) Date of publication of application:
**14.06.89 Bulletin 89/24**

(84) Designated Contracting States:
**DE ES FR GB IT NL SE**

(71) Applicant: **MILES INC.**
**1127 Myrtle Street**
**Elkhart,IN 46515(US)**

(72) Inventor: **Charlton, Steven C.**
**1533 Strong Avenue**
**Elkhart Indiana 46515(US)**
Inventor: **Denton, James B.**
**39 Harrison Avenue Apt. 24**
**Montclair New Jersey 07042(US)**
Inventor: **Makowski, Eugenia**
**19311 Wedgewood Drive**
**South Bend Indiana 46637(US)**

(74) Representative: **Dänner, Klaus, Dr. et al**
**c/o Bayer AG Konzernverwaltung RP**
**Patentabteilung**
**D-5090 Leverkusen 1 Bayerwerk(DE)**

(54) **Homogeneous aqueous solution electrolyte test.**

(57) The invention provides methods of producing a homogeneous aqueous solution capable of measuring the concentration of an ion in an aqueous sample and compositions useful for this purpose. The invention is particularly useful for measuring alkali cations of medical interest such as sodium, potassium, calcium and lithium. When a protein ladened body fluid, such as serum, is the test sample, the test composition or solution preferably includes a stabilizer such as a water miscible polymer or a detergent. The invention provides a single phase aqueous test solution which can be used on conventional analyzers including large clinical analyzers with other colorimetric diagnostic tests. No phase separation or centrifugation is required to perform the measurement.

EP 0 319 863 A2

## HOMOGENEOUS AQUEOUS SOLUTION ELECTROLYTE TEST

### I. INTRODUCTION

The invention relates to the measurement of the concentration of an ion in an aqueous sample in general and to the measurement of an electrolyte in body fluid sample, such as potassium in serum, in particular.

The measurement of serum potassium is a widely used and important diagnostic test. The measurement requires high sensitivity and precision since the total clinical range is only from about 2 to 10 millimolar (mM) with a normal range from about 3.5 to 5.5 mM. These considerations are also true of serum sodium measurements where the total range is 120-170 mM and the normal range is 135 to 155 mM. Measurement of lithium levels in serum is also important since the toxic dose level of lithium is only slightly higher than the therapeutic level used in psychiatric treatment. Other cations, such as calcium, sodium and magnesium, are also considered medically important. Although this invention is particularly significant for the measurement of alkali metal cations found in body fluid electrolytes, other ions, especially other cations, can also be measured.

### II. BACKGROUND OF THE INVENTION

At present, diagnostic tests for the determination of electrolytes such as sodium, potassium and lithium are routinely measured by flame photometry and ion selective electrodes. Recently two other methods have become available. Dry phase reagent strips are available for the colorimetric determination of serum potassium on a SERALYZER® reflectance photometer, both reagent strips and instrument are marketed by the Miles Diagnostics Division of Miles Inc., Elkhart, Indiana. The dry phase reagent strip technology is disclosed in U.S. Patent Nos. 4,540,520; 4,552,697; 4,649,123; 4,645,744; and 4,670,218.

Abbott Laboratories has marketed a specialized instrument and reagent package which measures serum potassium by partitioning a chromogenic counterion between liquid organic and aqueous phases. The organic phase contains an ionophore specific for potassium ion and the amount of chromogenic counterion partitioning into the organic phase is a function of potassium ion concentration. The instrument performs the required centrifugation of the reagent pack to cause phase separation and measures the chromogenic counterion concentration in the organic phase by absorbance. (See European Patent Applications 85105106.0 (published as EP 160282), 85105108.6 (published as EP 160901) and U.S. Patent No. 4,639,424.)

With the exception of flame emission photometry, most methods of measuring alkali metal cations with ionophores require the transport of the ion by the ionophore either into a liquid organic phase (phase transfer measurements), or through an ion selective membrane containing an ionophore (electrodes or optodes). In the dry reagent strip measurements, the ionophore is isolated in a hydrophobic environment, commonly provided by a polymeric component and the transport of the ion into the hydrophobic matrix, which contains two molecular species, an ionophore and reporter, produces a detectable colorimetric response.

An exception to the requirement of isolation of the ionophore in a hydrophobic or organic phase is the work of Feinstein et al., Proc. Nat. Acad. Sci. USA, 68(9) :2037-2041, (Sept. 1971). Feinstein et al disclose a study of the formation of a cationionophore complex in water with two fluorescent dyes, 1-anilino-8-naphthalene sulfonate (ANS) and 2-$p$-toluidinyl-6-naphthalene sulfonate (TNS) in the anionic form. The study involved ionophorous antibiotics such as valinomycin and the macrotetralide actions. The method used by the authors to produce the aqueous solutions tested is not clearly described. On page 2037, col. 2, lines 9-10, Methods and Materials, it is stated that solutions were made up to 2.0 ml and buffered at pH 7.4 by 10 mM Tris, HCl. Later on page 2038 in the footnotes, it was stated that the antibiotic stock solutions were made with acetone or ethanol and the organic solvent concentration in the fluorescence assays did not exceed 2.5%. The authors speculated that the observed increase in fluorescence depolarization could be due to the binding sites of the cation-ionophore complex becoming saturated, but also speculated that the depolarization could be caused by energy transfer between fluorophore molecules within a micelle or attributable to dye interaction with aggregates of cation-ionophore complexes.

Feinstein purposed to show that cation-ionophore complex could be detected in an aqueous environment. This was contrary to common wisdom that the stability of such complexes was drastically reduced by the addition of water to an organic solution containing the complexes (p. 2037, col. 2, lines 9-13).

Vogtle et al, U.S. Patent No. 4,367,072, disclose a change in color with the addition of an ion test sample when a hydrazinium hydrochloride is dissolved in methanol/water and mixed with molar amounts of a crown ether. A change of the absorption (brightening) is observed when the dye is displaced from its inclusion complex by a cation such as potassium or sodium.

Surface active properties of ionophores have been studied by Ping-Lin Kau et al, Tenside Detergents, 19(4) :204-206 (1982) and by J. Le Moigne et al, J. Phys. Chem., 84:170-177 (1980).

None of the tests described provides a homogeneous aqueous solution which can be used as a diagnostic test for clinically important electrolytes such as serum potassium.

## III. DESCRIPTION OF THE DRAWINGS

Each drawing is a dose response curve indicating the millimolar concentration of ion measured on the abscissa versus the optical density (absorbance) measured on the ordinate. It is apparent that in each case good linear results are obtained in the concentration range of clinical interest.

Fig. 1 shows the dose response of a sodium test solution containing only ionophore, reporter and buffer, (Example A1).

Fig. 2 shows the dose response of a homogeneous aqueous test solution for potassium to a spiked serum sample. The test solution was stabilized with ethylene/maleic anhydride copolymer added with the working buffer solution, Example A2a). Part b) of the Example shows that little, if any, reactivity is lost by lyophilization and reconstitution of the test solution.

Fig. 3 shows the dose response of a potassium test solution containing no organic solvent. A stabilizer was added with the working buffer solution, (Example A3).

Fig. 4 shows a dose response for a sodium test solution. A stabilizer was added to the organic reagent solution, (Example A4a).

Fig. 5 shows the dose response of the sodium test solution, prepared as that shown in Fig. 4 but with a different organic solvent, to sodium ion spiked serum samples, (Example A4b).

Fig. 6 shows the dose response of a potassium test solution, prepared by hydrolyzing a film, to normal serum spiked with potassium ion, (Example A5).

Fig. 7 shows the dose response of a potassium test solution. A stabilizer, Triton® X-100, was added to the organic reagent solution which was added directly to the working buffer solution to produce the test solution, (Example B3).

Fig. 8 shows a dose response for potassium ion with the addition of polyoxyethylene-9-lauryl ether as a stabilizer (Example B4).

Fig. 9 shows a dose response with a homogeneous aqueous sodium test solution prepared with a soluble polyurethane stabilizer as shown in Example B5.

Fig. 10 shows the dose response obtained with a centrifugation homogeneous aqueous test solution prepared with Pluronic® F-68 as the stabilizer, as shown in Example B6.

Fig. 11 shows the dose response obtained using phosphatidyl choline as a stabilizer (Example B7).

Fig. 12 shows a dose response to calcium ion with a homogeneous aqueous test solution prepared as shown in Example C1.

Fig. 13 shows the dose response to potassium ion with a homogeneous aqueous test solution containing valinomycin, prepared as shown in Example C2.

Fig. 14 shows the dose response to potassium ion using TBDE as the reporter, (Example D1), and

Fig. 15 shows the dose response to potassium ion using methyl eosin as the reporter, (Example D2).

## IV. SUMMARY OF THE INVENTION

The invention provides a homogeneous test solution capable of measuring ions present in Groups Ia and IIa of the Periodic Table of the Elements, such as potassium, sodium, calcium and lithium in aqueous solutions such as body fluid samples. The test solution contains an ionophore capable of forming a complex with an ion of interest; a neutral reporter having a dissociable proton which dissociation produces a detectable response; and a buffer capable of providing a pH in the pH range where deprotonization of the reporter occurs in response to the formation of the ionophore-ion complex. Preferred solution formulations include a stabilizer such as a water soluble polymer or water solubilizable polymer or a detergent.

3

The invention also provides several methods for preparing the aqueous test solution. In one embodiment the ionophore and neutral reporter can be dissolved in an aqueous solution having a pH which produces dissociation of the proton to form a solution and the pH of that solution can be adjusted to provide a test solution in the desired pH range. In another embodiment the ionophore and reporter can be dissolved in a water miscible organic solvent and steps for raising the pH and readjusting the pH to produce the final desired working pH are performed. In a preferred embodiment a stabilizer is added to the composition during one of the preparing steps. When a stabilizer is added to the composition; the ionophore, reporter and stabilizer can be dissolved directly into a buffer solution at the working pH. Adding the stabilizer when adjusting to the final desired working pH is particularly preferred. The test solution can also be prepared from water solubilizable polymer film containing ionophore and reporter or from lyophilized components by reconstitution.

## V. PREFERRED EMBODIMENTS OF THE INVENTION

Prior to the present invention, it was not believed possible to provide a sensitive, accurate ion test with an ionophore in a homogeneous aqueous solution. Although Feinstein et al purport to disclose the formation of the ionophore complex in aqueous solution, the solutions disclosed contained very high concentrations of ionophorous antibiotic in the crystalline form which easily dropped out of solution with gentle centrifugation. Although the concentration of analyte appeared to be very high, the Feinstein et al disclosure showed so little change in fluorescence in the clinical range of interest for potassium, the primary ion of interest, that one of ordinary skill in the art would be persuaded that the technique was not a viable avenue of research. Indeed, Feinstein et al state that no fluorescence changes were observed with synthetic "crown" ethers.

Therefore, most research continued in the vein of utilizing the ability of an ionophore to transport an ion either into an organic liquid phase, through a ion selective membrane or into a hydrophobic solid environment. During work with the solid phase ion test strips, researchers tried to increase the reactivity of nonporous film formats.

A key discovery which led to this invention was finding that preferred reporters, having a dissociable proton which are used in dry phase reagent strips, could be deprotonated to facilitate solubilization in an aqueous media and then protonated in the aqueous solution to provide the reactive species desired for the ion test.

After discoveries which indicated that conventional assumptions about the need for an organic or hydrophobic phase for a successful ion-ionophore test were erroneous, a broad spectrum of components and methods were discovered which provide a homogeneous aqueous test solution for ions. These test solutions are compatible with conventional analyzers including large scale, diagnostic analyzers which are based on the measurement of absorbance of an aqueous test solution to which a body fluid sample has been added.

## A. Test Components

### 1. IONOPHORE

Ionophores are well known in the art, although they have been generally thought to have minimal or low water solubility. Included in the term are neutral compounds which are electron rich and are capable of complexing cations. The ionophores contemplated include members of the classes of podands, cryptands and coronands as well as ionophorous antibiotics such as valinomycin. The coronand class referred to as crown ethers are particularly suitable. Particularly preferred ionophores are listed immediately prior to the Examples.

However, this invention is generally applicable to all members of the class of compounds generally known as ionophores. Numerous examples of ionophore/ion pairs can be found in the literature, see especially Simon and Pressman. Ionophores are capable of complexing ions and transporting them through lipid membranes or into an organic phase. The latter property is used to distinguish the class from the broader class known as "chelates". Chelates, while technically a broader classification, are commonly thought of as charged, water soluble molecules capable of complexing ions.

## 2. REPORTER

The reporter is a separate molecular species from the ionophore which can provide a detectable response upon formation of the ionophore-ion complex. A colorimetric response is preferred although a fluorometric response can be used with suitable measuring equipment. Reporters useful in this invention are neutral compounds having a dissociable proton. With suitable pH conditions, which will be discussed later, the proton dissociates upon formation of the ionophore-ion complex producing the detectable response. The reporters can be referred to as having an acidic proton. Preferred reporters contain an aromatic hydroxyl, a proton which can dissociate to produce a detectable response. Two classes of indophenol reporters have been described in U.S. Patent Nos. 4,540,520 and 4,552,697, which are expressly incorporated by reference herein. However, other classes of reporters are also suitable including triphenylmethanes such as tetrabromophenolphthalein decyl ester (TBDE) and fluoresceins such as methyl eosin.

Reporters which have a hydrophilic portion and a hydrophobic portion are preferred. Particularly preferred is the indophenol, 7-(n-decyl)-2-methyl-4-(3´,5´-dichlorophen-4´-one)-indophenol, referred to herein as 7-decyl MEDPIN. The increased stability and ease of preparing the desired aqueous test solution with these compounds may be linked to a possible micellar structure of the test solution.

## 3. BUFFERING SUBSTANCE

It has been found that the reporter provides a detectable response upon formation of the ionophore-ion complex by loss of a proton. The buffering substance must have a pH which allows this mechanism to take place and which inhibits proton loss from purely pH or environmental effects. Usually the working pH range desired is between about 5 to 10, but the choice will depend on the test components, the test sample and even on the desired sensitivity of the test. At a high pH, proton loss occurs more readily and provides an apparent increase in reactivity. At lower pH the inverse is true. The working pH range will provide a neutral, essentially protonated, reporter species prior to contact with the ion of interest. In the preferred colorimetric reporters, the state of the reporter is often dramatically evident since the color of the test solution will often change radically. The determination of a suitable pH is a routine laboratory experiment given the disclosure in the Examples.

## 4. STABILIZER

The stabilizer appears either to maintain the reporter and ionophore in aqueous solution or to actually help solubilize these components. Useful stabilizers appear to be those with surface active properties and include water soluble, or water solubilizable polymers, and compounds which are commonly referred to as detergents. A water solubilizable polymer is one which is insoluble in water, but after some simple reaction becomes water soluble. A wide variety of components have been found to be suitable. Useful polymers include, but are not limited to, poly(vinyl alcohol), polyvinylpyrrolidone, Pluronic® block polymers such as polyethylene oxide/polypropylene oxide polymer, and copolymer anhydrides such as ethylene/maleic anhydride, butylene/maleic anhydride, and methylvinyl ether/maleic anhydride, known as GANTREZ® and supplied by GAF Corp. The Pluronics and anhydrides are preferred. Other useful compounds include phosphatidyl choline and detergents such as nonionic surfactants, e.g., Triton® X-100, Tweens, Brij 35, and polyoxyethylene-9-lauryl ether (polidocanol). The use of a stabilizer, while optional, is preferred.

## B. Aqueous Test Solution

As will be evident from the examples and the description of the methods of preparing the test solution, the aqueous solution can be either solely water, containing the above components, or can also contain a small amount of a water miscible organic solvent. The water miscible organic solvent can be ethanol, methanol, tetrahydrofuran, acetone and the like. The concentration is usually less than 4%. A high concentration of organic solvent is not preferred since its rapid evaporation could cause the concentration of the test solution to vary appreciably on standing. In addition, the use of organic solvents with clinical analyzers may cause deterioration of the tubing, therefore creating problems for the user.

## C. Concentration Ranges of Test Components

The concentrations of the test components are not critical to the invention provided that the concentrations of the ionophore, reporter and buffer are sufficient to produce the desired detectable response. For qualitative results neither the concentration of the ionophore nor the concentration of the reporter is tied to the concentration range of the ion to be determined.

Determination of the optimum concentration of components is within the ability of one skilled in the art, given the present disclosure. However, the following guidelines are provided. The concentration of organic solvent is usually kept to a minimum, generally less than 4%. The concentration of polymer stabilizers can vary widely, but the concentration of detergent stabilizers is generally less that 0.5%. Detergent stabilizer concentrations above this level appear to produce anomalous results, perhaps due to the formation of mixed micelles.

## D. Method of Preparing the Test Solution

After the discovery that it was possible to prepare an aqueous test solution with ionophores which was reactive to ions, it was found that there were many methods by which such solutions can be prepared. However, it was found that the most straight forward methods, as used by Feinstein et al, do not produce a homogeneous test solution. Neither dissolving the reactive reagents, ionophore and reporter directly in a buffer having a suitable working pH; nor dissolving the reactive reagents in a water miscible organic solvent and then adjusting the pH with a buffer having the desired working pH, thereby also diluting the organic solution to produce a test solution containing less than 4% by volume organic solvent, produces a reactive homogenous test solution capable of measuring the concentration of ions at clinically significant levels. However, several viable methods are outlined below. The phrase "reactive reagents" is used herein to refer to the ionophore and the neutral reporter species.

### 1. Method Requiring Dissolution in an Organic Solvent

There are several suitable methods in which the dissolution of the ionophore and reporter in a water miscible organic solvent is the first step to preparing a homogeneous aqueous solution. The reactive reagents, can be dissolved in a water miscible organic solvent. The organic solution thus formed is then added with stirring to a basic aqueous solution having a pH high enough to cause dissociation of the reporter. This dissociation is usually distinct with colorimetric reporters since the color of the dissociated form is commonly very different from the color of the neutral form. The pH of the high pH solution is then adjusted by adding an aliquot of the solution containing dissociated reporter to a buffer solution having a pH in the range wherein the reporter is in the neutral form and the dissociation of the reporter will now occur with formation of the ionophore-ion complex, not simply due to the pH of the sample. The pH range where the dissociation of the reporter takes place in response to the formation of the ionophore - ion complex is referred to herein as the "working pH range".

A second and preferred method is to add a stabilizer. The stabilizer can be dissolved in the organic solvent with the reactive reagents, in the basic aqueous solution or added with the solution used in the final pH adjusting step. Of these methods, the use of a stabilizer in the final pH adjusting step, dissolved in a buffer solution, is preferred.

When a stabilizer is used it is also possible to produce the aqueous test solution by immediately adding the organic solution, or an aliquot thereof, to a buffer solution having the desired final pH, with stirring. In such a method it is preferred to use a stabilizer in the organic solution. The water miscible organic solvent can be chosen from any of the solvents which will dissolve the reactive reagents in the desired concentrations. Tetrahydrofuran (THF), acetone, methanol and ethanol have all been used with success, although for simple solubility reasons THF has been preferred.

The degree of stirring can be important since the reactive reagents are not very soluble in water. Occasionally, with any of the methods disclosed herein, some particulate matter remains in the test solution with bench made solutions. Often it is such a small amount that it can be ignored. However, any particulate matter, which has been found to be ionophore and reporter, which was not completely dissolved, can be removed by mild centrifugation ($0.1 \times 10^6$ g-mins) and the homogeneous test solution remaining is reactive to ion at desired concentration levels. The bulk of the reactive reagent remains in solution even after prolonged centrifugation ($1 \times 10^6$ g-mins). Similar experiments carried out with an aqueous solution made by the method of Feinstein et al produced nonreactive solutions after only very mild centrifugation (0.01 x $10^6$ g-mins), suggesting that the ionophore is present in particulate form.

2. Methods Requiring No Organic Solvent.

Other methods do not require dissolution of the reactive reagents in water miscible organic solvent. It is possible to dissolve the reactive reagents directly into a high pH solution, having a pH capable of dissociating the proton of the reporter, with stirring, and then adjusting the pH of that high pH solution to the desired working pH range with an aqueous buffer solution. Each solution produced, of course, contains a decreasing amount of reactive reagents. Therefore, the concentration used initially should be high enough that the desired concentrations are reached upon dilution with the buffer at the final working pH.

Improved stability of the test solution produced is obtained when a stabilizer, preferably a polymer stabilizer, is added to the reagents at some point in the preparation of the test solution. Addition with the initial dissolution step is preferred. It is speculated, although not relied upon herein, that the stabilizer either promotes the solubilization of the reactive reagents by participating in micelle formation; or stabilizes micelles of the reactive reagents once formed.

A polymer film containing the reactive reagents can also be dissolved directly to produce a homogeneous test solution having the desired working pH. The polymer film can be formed either by lyophilizing any of the test solutions made above containing a polymer stabilizer or by drying by any other method which would produce such a film. The film can also be produced as disclosed in U.S. Patent No. 4,645,744, as long as the hydrophobic vehicle used is water soluble or water solubilizable. Films formed with polymeric anhydride components such as ethylene/maleic anhydride copolymer, by the latter technique, are preferred. Films made from an ionophore, neutral reporter and polymeric anhydride, which apparently acts as a stabilizer, are dissolved by hydrolyzing the film. The film is usually contacted with a buffer solution having the desired final pH and heated to hydrolyze and/or dissolve the film. Heating such films to about 50°C for ten minutes with buffer has been sufficient to effect solution.

Each of these methods produces a homogeneous aqueous test solution capable of measuring the concentration of the ion for which the ionophore is specific. The methods used inherently decrease the concentration of the reactive reagents present initially. Therefore this dilution effect must be taken into account when the initial concentrations of reactive reagents is calculated. Guidance for preparing test solutions having concentrations useful for testing cations of clinical interest can be found in the Examples.

E. Utility

Homogeneous aqueous test solutions are useful for the determination of many ions. They are particularly preferred for the determination of clinically important cations and especially those found in Groups Ia and IIa of the Periodic Table of the Elements. The test solutions can be sold as the final test solution, as solutions to be diluted or the test components can be sold as a film or in the dry state to be dissolved.

These test solutions have particular utility for ion measurement of body fluids with liquid clinical analyzers commonly used. In order to use the aqueous test solutions with samples containing large amounts of proteins, such as serum samples, it is preferred to keep the concentration of organic solvent low, due to the possible precipitation of proteins. In addition, the use of a stabilizer with the reactive reagents is preferred with serum samples. The presence of large amounts of serum proteins appears to "destabilize" the test solution and produce precipitation of the reactive reagents unless a stabilizer is present.

VI. ABBREVIATIONS AND STRUCTURES

The following abbreviations are used in the Examples which follows.

Square brackets, [ ], can designate ion concentration in millimoles per liter (mM). All percent concentrations are given in volume per deciliter (% v/v) unless otherwise indicated. All reporters used are in the neutral form originally and in the final test solutions produced.

| g-min | g force applied for one minute |
| OD | optical density (absorbance) |
| Temperature: | |
| $^\circ$C | degrees Centigrade |
| Weight: | |
| gm | gram |
| mg | milligram |
| Volume: | |
| dL | deciliter |
| mL | milliliter |
| L | liter |
| Concentration: | |
| mM | millimolar (millimoles per liter) |
| M | molar (moles per liter) |
| % w/v | percent weight per deciliter |
| % v/v | percent volume per deciliter |
| Ions: | |
| $Na^+$ | sodium ion |
| $K^+$ | potassium ion |
| $Li^+$ | lithium ion |
| $Ca^{++}$ | calcium ion |

Abbreviations for chemical components used are given below. The ionophore designations were assigned by the present inventors for convenience only. The name is usually based on the principal ion the ionophore is used to determine. However, ionophores commonly respond, to varying degrees, to other ions. (Struc tures of preferred ionophores can be found in U.S. Patent No 4,670,218).

Ionophores

| | | |
|---|---|---|
| | Sodium Ionophore | *bis*[12-crown-4-methyl] methyldodecylmalonate |
| | Potassium Ionophore | 2,3-naphtho-1,4,7,10,13-pentaoxacyclopentadeca-2-ene |
| | Lithium Ionophore I | N,N'-diheptyl-N,N'-5,5-tetramethyl-3,7-dioxanonane diamide |
| | Lithium Ionophore II | N,N'-diheptyl-5,5-dimethyl-N,N'-di-(3-oxapentyl)3,7-dioxanonane diamide |
| | CDA (For lithium) | *cis*-N,N,N',N'-tetra-isobutyl-1,2-cyclo-hexane dicarboxamide |
| | Calcium Ionophore | diethyl-N,N'-[(4R,5R)-4,5-dimethyl-1,8-dioxo-3,6-dioxaocta-methylene]*bis*(12-methyl-aminododecanoate) |

Stabilizers

| | | |
|---|---|---|
| | EMA | ethylene/maleic anhydride copolymer (Aldrich Chemical Co. Catalog No. 18,805-0) |
| | Gantrez® | methylvinyl ether/maleic anhydride copolymer (from GAF Corp.) |
| | BMA | butylene/maleic anhydride |
| | PVA | poly(vinyl alcohol) (Aldrich Chemical Co. Catalog No. 18,463-2) |

9

| PVP | polyvinylpyrrolidone (Aldrich Chemical Co. Catalog No. 85,656-8) |
| --- | --- |
| Triton® X-100 | polyethylene glycol-$p$-isooctylphenyl ether (Sigma Chemical Co., Catalog No. T-6878) |
| Pluronic® F68 | ethylene oxide/poly-propylene glycol conden-sation product (BASF Wyandotte Corp.) |
| Brij® 35 | [polyoxyethylene (23) lauryl ether] (ICI United States, Inc., Wilmington, DE) |
| Polidocanol | polyoxyethylene-9-lauryl ether (Sigma Chemical Co. Catalog No. P9641) |

Reporter
| 7-decyl MEDPIN | 7-($n$-decyl)-2-methyl-4-(3',5'-dichlorophen-4'-one)-indonaphthol |
| --- | --- |
| TBDE | tetrabromophenol-phthalein decyl ester |
| Methyl eosin | methyl(tetrabromo fluorescein) (Aldrich Chemical Co. Catalog No. 19,955-9) |

**Buffering Substance**

| Bis-Tris | $bis$[2-hydroxyethyl]-imino-$tris$(hydroxy-methyl)methane |
| --- | --- |
| Bis-Tris propane | 1,3-$bis$[$tris$(hydroxy-methyl)methylamino]-propane |
| Tris | $tris$(hydroxymethyl)-aminomethane |

| PIPES | piperazine-N,N'-bis[2-ethanesulfonic acid] |
| HEPES | (N-2-hydroxyethylpiper-azine-N',2-ethane-sulfonic acid) |
| Bicine | N,N-*bis*[2-hydroxyethyl] glycine |
| MES hydrate | (4-morpholine ethane sulfonic acid) |

Miscellaneous

| THF | tetrahydrofuran |
| KCl | potassium chloride |
| NaOH | sodium hydroxide |
| LiOH | lithium hydroxide |
| Kodar A-150 | (polycyclohexylene dimethylene tere-phthalate, acid modified) |

## VII. EXAMPLES

A general method for preparing a homogeneous test solution is as follows:

a) Dissolve an ionophore and a neutral reporter in a water miscible organic solvent to produce an organic reagent solution;

b) Add the organic reagent solution, dropwise, to a vortexed basic solution to produce a high pH reagent solution where the reporter is in the deprotonated form; and

c) Add the high pH reagent solution to a working pH buffer solution to produce a homogeneous aqueous test solution where the reporter is essentially in the nonionized form.

The organic solvent used was THF and the reporter was 7-decyl MEDPIN, unless noted otherwise. The dose response was at 640 nanometers (nm) unless noted otherwise and concentration of the ion shown is that in the cuvette.

### EXAMPLE A: METHODS

#### Example A1: Sodium Test Solution

A homogeneous test solution for sodium was prepared as described in the general method. *Bis*[12-crown-4-methyl]methyldodecylmalonate, an ionophore referred to herein as the "sodium ionophore", was used. The organic reagent composition was: sodium ionophore, 17 mM, and 7-decyl MEDPIN, 33.6 mM. This solution was red. The basic solution was lithium hydroxide (LiOH), 25 mM, and the high pH reagent solution produced was blue with the reagent concentration 1/10 of the original values. The working pH

11

buffer solution contained 250 mM HEPES adjusted to pH 6.5 with LiOH. The homogeneous test solution was clear orange and contained 3.3% (v/v) THF, sodium ionophore, 0.567 mM, reporter, 1.12 mM, and buffer, 167 mM.

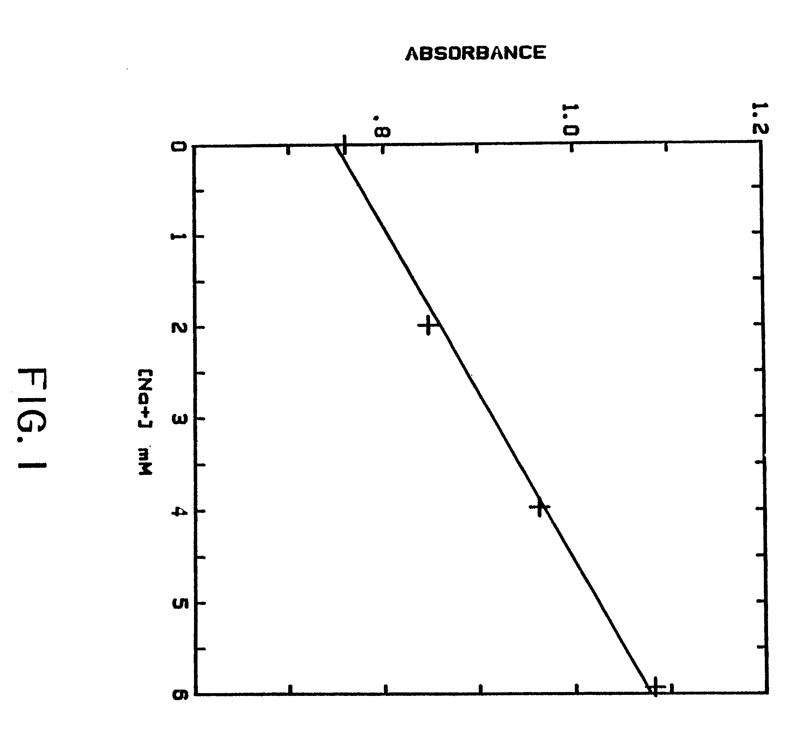

The test solution prepared was tested with aqueous sodium solutions. The data are given below and shown in Fig. 1

| $[Na^+]$ (mM) | OD |
|---|---|
| 0 | .760 |
| 1.99 | .847 |
| 3.97 | .963 |
| 5.93 | 1.083 |

The homogeneous aqueous test solution prepared showed a good response to sodium at physiological levels.

Example A2: Potassium Test Solution

A homogeneous test solution for potassium was prepared as described in the general method with a stabilizer added with the working buffer solution.

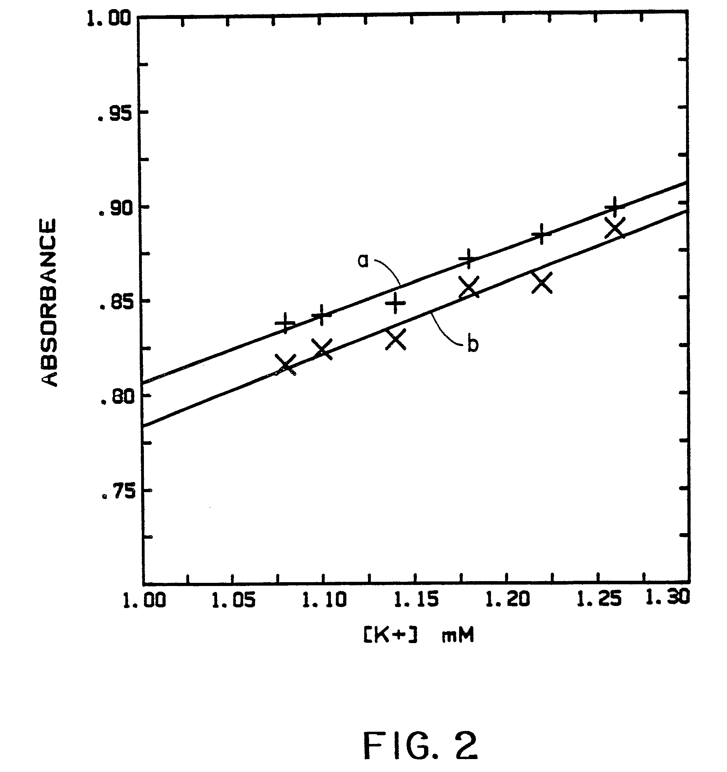

a) 2,3-naphtho-1,4,7,10,13-pentaoxacyclopentadeca-2-ene, an ionophore referred to herein as the potassium ionophore was used. The organic reagent composition was potassium ionophore, 43 mM, 7-decyl MEDPIN, 33.6 mM. The organic reagent solution was red. The basic solution was 50 mM NaOH and the high pH reagent solution produced was blue, with reagent concentrations 1/10 of the original values. The working pH buffer solution was 1M HEPES, pH 6.5, containing 1% w/v of the stabilizer, ethylene/maleic anhydride copolymer. A clear orange homogeneous aqueous test solution resulted containing THF 3.3% v/v, potassium ionophore, 1.43 mM, reporter 1.12 mM, stabilizer, 0.67% w/v, buffer, 670 mM. A dose response curve was generated with this test solution with a potassium spiked normal human serum sample: 0.8 mL test solution was added to 0.2 mL sample. The data obtained is shown after part A2b, below. See Fig. 2(a)

b) An aliquot of the test solution prepared in part A2a was lyophilized. Several days later it was reconstituted to the original volume with water while stirring. A small amount of residual solid material was removed by centrifuging ($0.3 \times 10^6$ g mins) and the supernatant tested with potassium spiked normal human serum as described above. The results are shown below. It is apparent that little, if any, reactivity was lost by lyophilization and reconstitution. The data obtained is shown below. See Fig. 2(b).

| $[K^+]$ (mM) | (a) OD | (b) OD |
|---|---|---|
| 1.08 | .838 | .816 |
| 1.10 | .842 | .824 |
| 1.14 | .848 | .829 |
| 1.18 | .871 | .856 |
| 1.22 | .884 | .858 |
| 1.26 | .898 | .887 |

Example A3: Potassium Test Solution

A potassium test solution was prepared without dissolving the reactive reagents in an organic solvent. 7-decyl MEDPIN (7.47 mM) was dissolved in basic solution (NaOH, 50 mM). The potassium ionophore (15

12

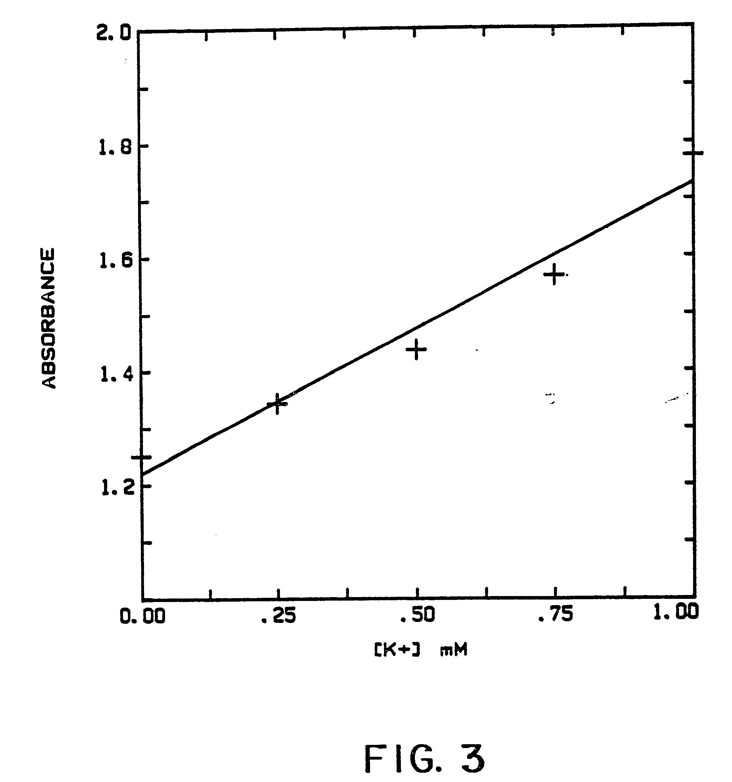

mM) was then dissolved into the basic solution to generate a high pH reagent solution (sonication was used to speed the process). The working pH buffer solution was HEPES, (1M, pH 6.5) containing 1% w/v of the stabilizer ethylene/maleic anhydride copolymer. A somewhat muddy brown solution containing potassium ionophore 5 mM, reporter, 2.49 mM, buffer, 667 mM, and stabilizer, 0.67% w/v, resulted. It was clarified with a 0.45 μ filter to give a clear orange liquid which was tested with aqueous potassium sample. The data are shown below and in Fig. 3.

| $[K^+]$ (mM) | OD |
|---|---|
| 0 | 1.251 |
| 0.25 | 1.344 |
| 0.5 | 1.437 |
| 0.75 | 1.566 |
| 1.0 | 1.776 |

The homogeneous aqueous test solution, containing no organic solvent, showed good response to potassium ion in the concentration range of clinical interest.

Example A4: Sodium Test Solution

A homogenous test solution for sodium was prepared with the addition of a stabilizer in the organic reagent solution.

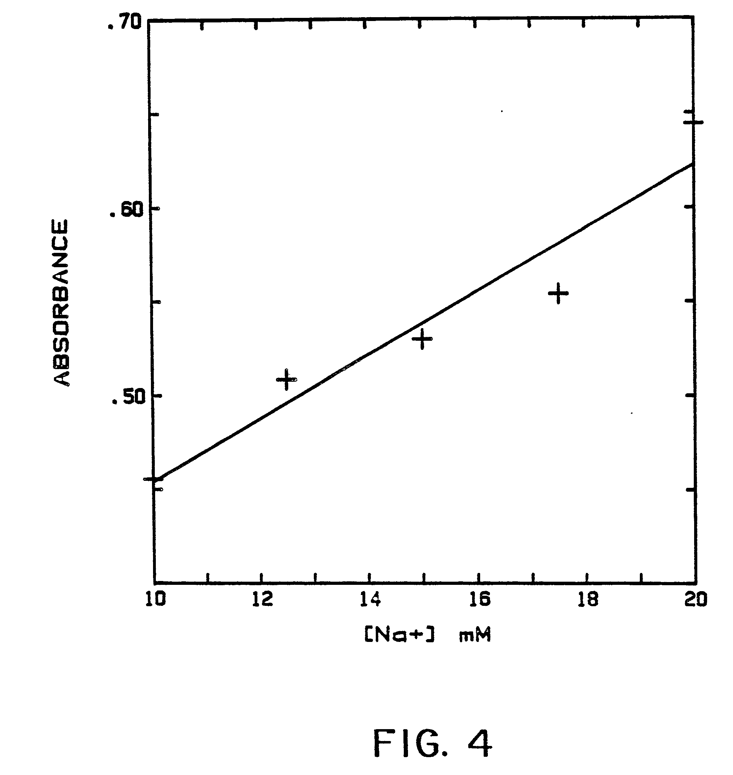

a) The organic reagent solution contained: sodium ionophore, 37.7 mM, 7-decyl MEDPIN, 33.6 mM, and stabilizer ethylene/maleic acid copolymer, 10% w/v. This solution was red and was added dropwise directly to the vortexed working pH buffer solution. The buffer solution was composed of 1M HEPES, adjusted to pH 6.5 with LiOH. The homogeneous test solution produced was clear orange and contained THF, 3.3% v/v, ionophore, 1.26 mM, reporter, 1.12 mM, buffer, 970 mM, and stabilizer, 0.33% w/v. The dose response of the test solution was measured after the addition of a aqueous solution containing sodium ion aliquot and is shown in Fig. 4.

| $[Na^+]$ (mM) | OD |
|---|---|
| 10 | .4557 |
| 12.5 | .5086 |
| 15 | .5300 |
| 17.5 | .5539 |
| 20 | .6443 |

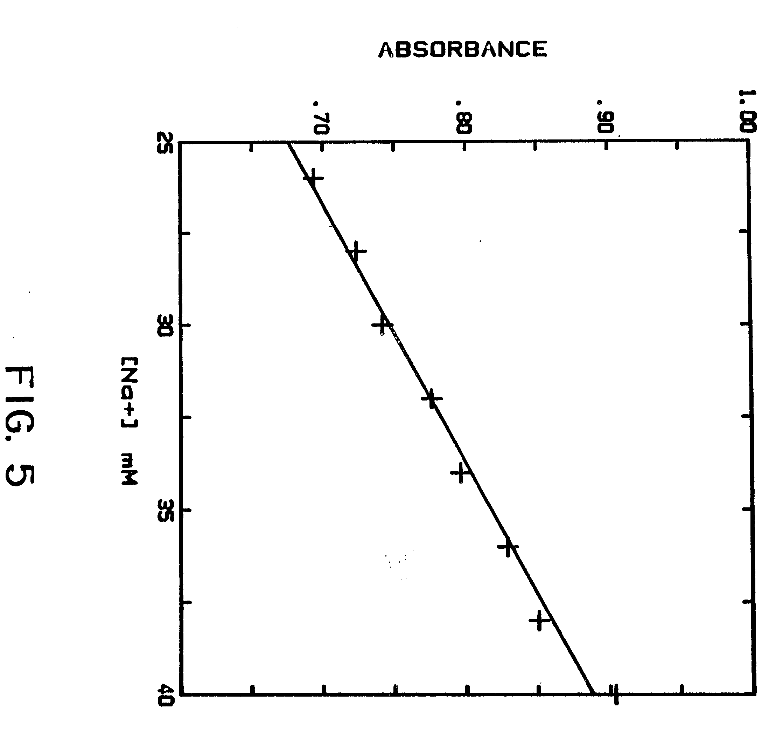

b) The test solution was prepared and tested as described in example A4a, except that acetone was used as the organic solvent. Aliquots of human serum were spiked to produce samples with sodium ion concentrations ranging from 130 to 200 mM. The test solution (0.8 ml), was mixed with the spiked serum sample, (0.2 ml) to produce the dose response shown in Fig. 5.

| [Na$^+$] (mM) | OD |
|---|---|
| 26 | .694 |
| 28 | .724 |
| 30 | .742 |
| 32 | .776 |
| 34 | .796 |
| 36 | .829 |
| 38 | .851 |
| 40 | .904 |

These experiments show that stabilizer can be added in the organic reagent solution and that acetone can be used as a organic solvent as well as THF. The stabilized homogeneous aqueous test solution prepared was responsive to the concentration of sodium ion in the physiological fluid, serum.

### Example A5: Test Solution Prepared from a Solubilizable Film

A potassium test solution was prepared from a film. A film was prepared from an organic reagent solution similar to Example A4 containing potassium ionophore, 143.1 mM, 7-decyl MEDPIN, 33.6 mM, and ethylene/maleic anhydride copolymer, 10% w/v, in THF. This solution was spread onto Kodar A-150, to a thickness of $10 \times 10^{-3}$ inches (250 $\mu$). The THF was allowed to evaporate, leaving a nonporous orange polymer film.

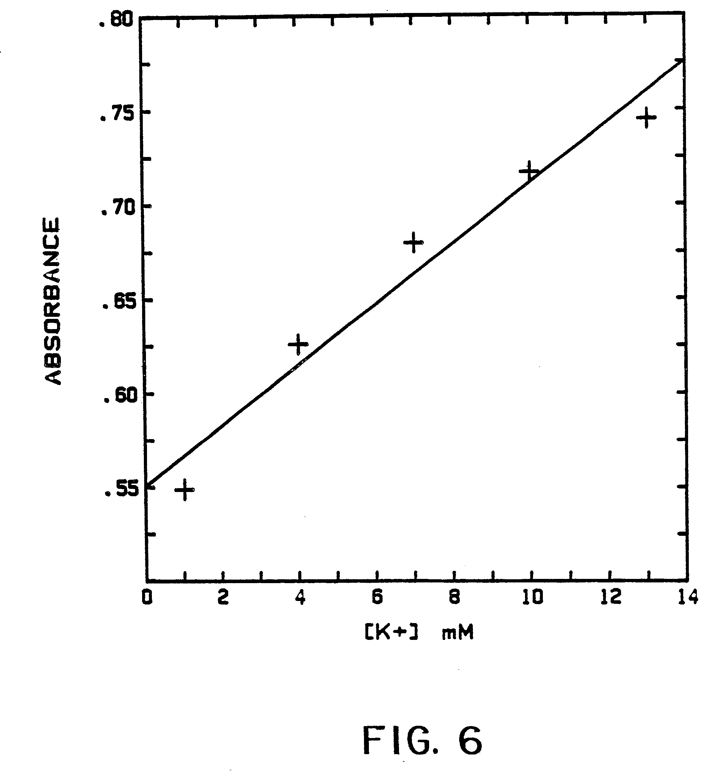

A one inch (2.5 cm) square piece of the material was immersed in HEPES buffer (1M, pH 7.5, 5 ml) and soaked for 10 minutes at 50° C with stirring. The resulting cloudy orange solution (0.75 ml) when tested with potassium spiked normal serum samples (0.75 ml) showed a response measured by absorbance, 3 minutes after mixing. The data obtained is given below and shown in Fig. 6.

| [K$^+$] (mM) | OD |
|---|---|
| 1.02 | .549 |
| 4.02 | .626 |
| 7.02 | .680 |
| 10.02 | .717 |
| 13.02 | .745 |

### Conclusions

i) A homogeneous test solution (Example A1) containing just ionophore, neutral reporter and buffer can be prepared which has a good response to sodium at physiological levels.

ii) Stabilized homogeneous test solutions were prepared responsive to sodium (Example A4) and potassium (Example A2) in physiological fluids.

iii) When a stabilizer is desired it can be included in any of the solutions (Example A2a, A4).

iv) The homogeneous solution can be reconstituted either from dried materials (Example A2b), or from a solubilizable film (Example A5).

v) The organic solvent is not essential (Example A3), but when used can be THF (Example A1) or other water miscible solvent, e.g., acetone (Example A4b).

### Example B: Variation in Stabilizer

14

### Example B1: Poly(vinyl alcohol) as a Stabilizer

A sodium test solution was prepared as described in the general method with the addition of a stabilizer in the working buffer solution. The organic reagent solution contained sodium ionophore, 37.7 mM, and 7-decyl MEDPIN, 33.6 mM. The solution was red. The basic solution was LiOH, 50 mM, and the high pH reagent solution was blue with reagent concentrations 1/10 of the original values. The working pH buffer solution was HEPES, 200 mM, pH 6.5 containing 1% w/v of poly(vinyl alcohol) (88% hydrolyzed, average molecular weight 10,000). A clear red homogeneous test solution was produced which contained THF, 3.3% v/v, sodium ionophore, 1.26 mM, 7-decyl MEDPIN, 1.12 mM, stabilizer, 0.67% w/v, and buffer, 133 mM.

With the addition of sodium chloride (100 mM), the solution turned blue.

### Example B2: Polyvinylpyrrolidone as a Stabilizer

A sodium test solution was prepared as described in Example B1 except with polyvinylpyrrolidone (average molecular weight 10,000) as a stabilizer. The same results were obtained.

### Example B3: Triton X-100 as Stabilizer

A potassium test solution was prepared with the stabilizer in the organic reagent solution as in Example A4a. The composition of the organic reagent solution was potassium ionophore, 71 mM, 7-decyl MEDPIN, 16.9 mM, and Triton X-100, 2.7% v/v. This solution was added directly to the working pH buffer solution, which was Bis-Tris, 100 mM, pH 6.5. The result was a fairly clear orange solution which contained THF, 4% v/v, ionophore, 2.86 mM, reporter 0.676 mM, buffer, 96 mM, and stabilizer, 0.108% v/v.

| $[K^+]$ (mM) | OD |
|---|---|
| 0 | .577 |
| 0.10 | .590 |
| 0.30 | .610 |
| 0.99 | .671 |
| 3.94 | .906 |
| 6.87 | 1.089 |

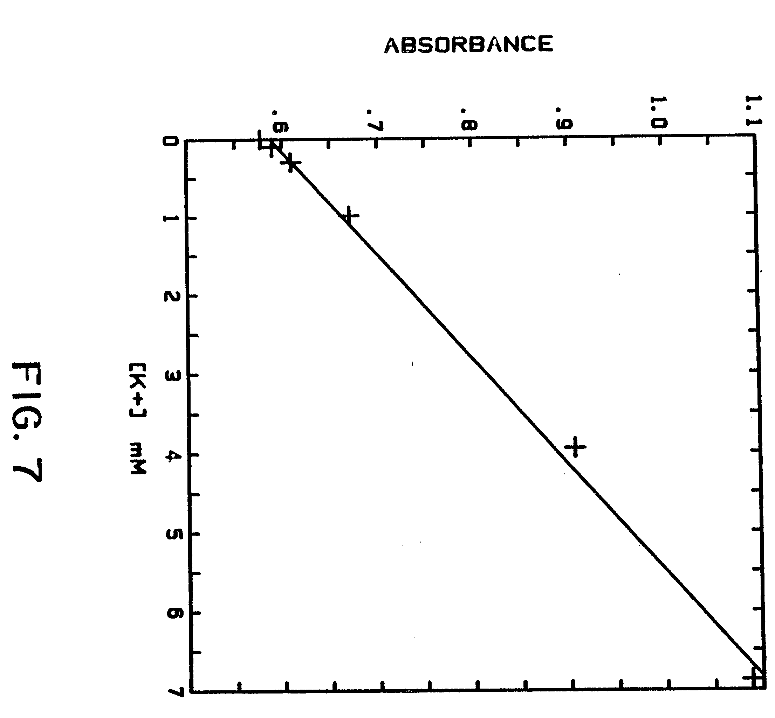

The test solution showed a good linear response to potassium as can be seen in Fig. 7.

### Example B4: Polyoxyethylene-9-lauryl Ether as a Stabilizer

A potassium test solution was prepared as described in the general method with the addition of a stabilizer in the working buffer solution. The organic reagent solution contained potassium ionophore, 42 mM, and 7-decyl MEDPIN, 33.8 mM. The basic solution was NaOH, 50 mM, and the high pH reagent solution produced was blue with reagent concentrations 1/3 of the original values. The working pH buffer solution was PIPES, 100 mM, pH 6.5, containing 0.1 % w/v polyoxyethylene-9-lauryl ether. The result was a clear homogeneous purple test solution containing THF, 2.8% v/v, ionophore, 1.17 mM, reporter, 0.936 mM, buffer, 89 mM, and stabilizer, 0.089% w/v. A dose response for potassium ion was measured at 600 nm.

| [K+] (mM) | OD |
|---|---|
| 0 | .856 |
| 0.19 | .918 |
| 0.38 | .982 |
| 0.57 | 1.036 |
| 0.76 | 1.102 |
| 0.95 | 1.161 |
| 1.14 | 1.212 |
| 1.33 | 1.266 |
| 1.52 | 1.316 |

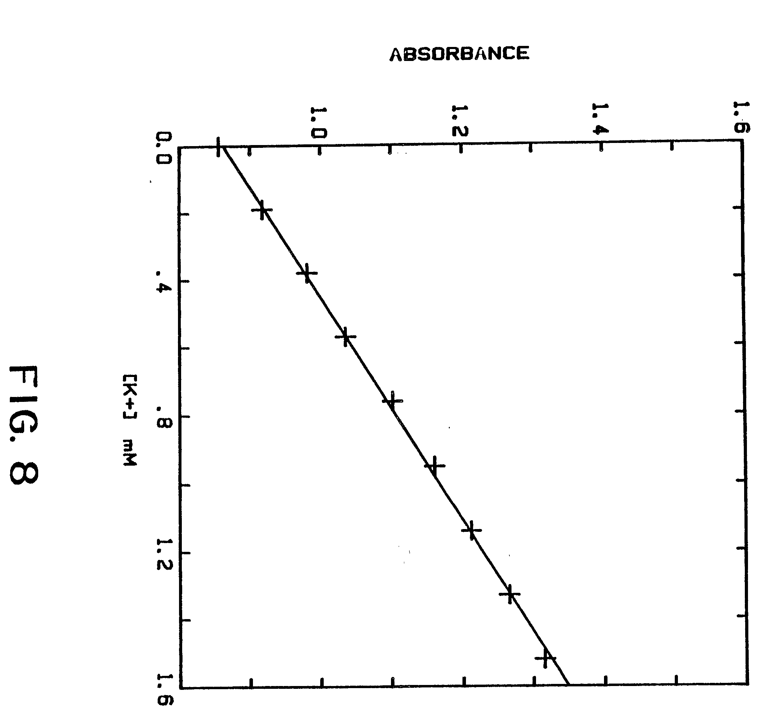

Fig. 8 shows the dose response obtained.


Example B5: Water Soluble Anionic Polyurethane Stabilizer


A sodium test solution was prepared as described in the general method with the addition of stabilizer in the buffer solution. The organic reagent composition was sodium ionophore, 226 mM, and 7-decyl MEDPIN, 200 mM. The basic solution was LiOH, 50 mM, and the reagent concentrations in the high pH reagent solution were 1/11 of the original values. HEPES, 500 mM adjusted pH 6.5 with LiOH, was used to prepare the working pH buffer solution with the addition of 1% of a soluble polyurethane stabilizer. Composition of the resulting solution was THF, 0.7% v/v, sodium ionophore, 1.6 mM, reporter, 1.4 mM, buffer, 462 mM, stabilizer, 0.92% w/v. This solution was centrifuged for $0.1 \times 10^6$ g-min. There was a small pellet but the supernatant was orange-red and was used for testing. The preparation was evaluated with aqueous sodium samples and with one spiked serum sample.

| [Na+](mM) | OD |
|---|---|
| 0 | .316 |
| 5 | .440 |
| 9.9 | .568 |
| 11.9 | .615 |
| 13.8 | .663 |
| 15.8 | .705 |
| 17.7 | .743 |
| 19.6 | .787 |
| 23.9 | .919* |

*Addition of serum

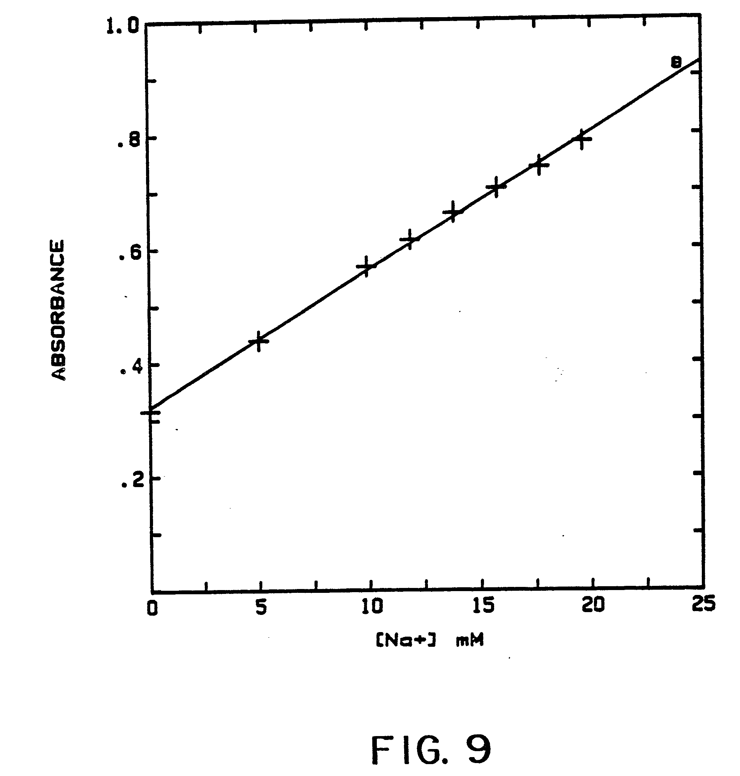

Fig. 9 shows the dose response obtained.


Example B6: Pluronic® F68 as a Stabilizer


A sodium test solution was prepared as described in the general method with the addition of a stabilizer.

a) The organic reagent solution contained sodium ionophore, 45 mM, and 7-decyl MEDPIN, 45 mM. The basic solution was LiOH, 25 mM. The reagent concentrations in the high pH reagent solution were 1/10 of the original values. HEPES, 500 mM, pH 7.5, was used to prepare a working buffer solution containing 1% Pluronic® F68. A clear brown solution resulted containing, THF 3.3% v/v, sodium ionophore, 1.5 mM, reporter, 1.5 mM, buffer, 333 mM, stabilizer, 0.67% w/v.

b) An aliquot was centrifuged ($1 \times 10^6$ g min.). There was no pellet. The dose response of the preparation both centrifuged and uncentrifuged was determined with serum samples, adding 0.05 ml sample to 0.95 ml of reagent.

| [Na$^+$] (mM) | (a) OD Uncentrifuged | (b) OD Centrifuged |
|---|---|---|
| 6.21 | 1.263 | 1.244 |
| 8.08 | 1.334 | 1.348 |
| 9.97 | 1.438 | 1.476 |

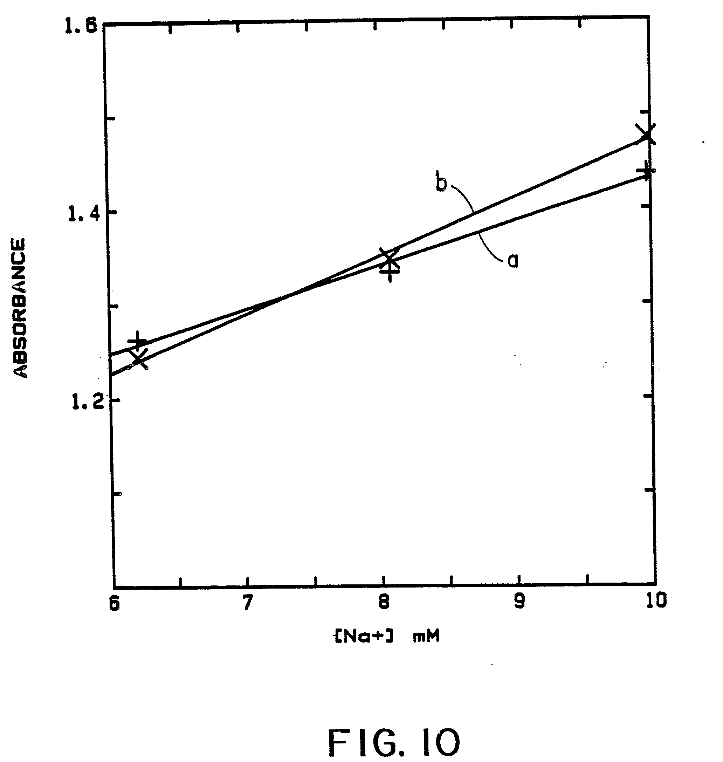

Fig. 10 shows good linear correlation for both centrifuged and uncentrifuged test solutions.

Example B7: Phosphatidyl Choline (as Liposomes) as a Stabilizer

a) Preparation of phosphatidyl choline Liposome. Concentrated L-α-phosphatidyl choline (1 mL, Sigma, 100 mg/ml in chloroform) was dried under vacuum to remove the chloroform. PIPES buffer (4 mL, 0.1M, pH 6.5) was added to the dried lipid film and the mixture was stirred until it formed a milky white suspension. The suspension was titrated with 10% sodium cholate until it became clear. The total volume of this clear lipid/detergent micelle solution was about 5 mL. The solution was dialyzed against 0.1 M PIPES buffer at pH 6.5 for 15 hours to remove sodium cholate. The buffer was changed twice during the dialysis. Clear phosphatidyl choline liposome solution (pale yellow color) was removed from the dialysis sac.

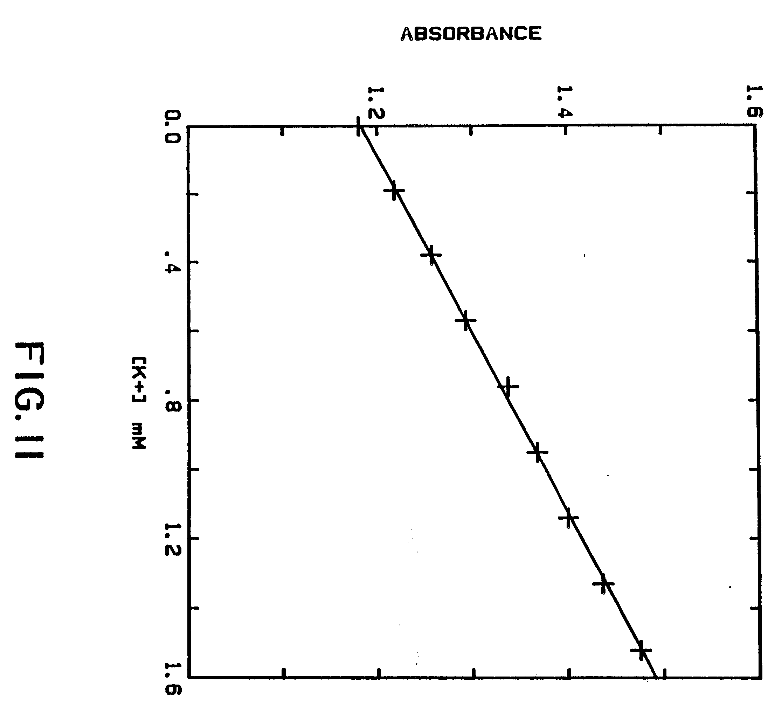

b) Preparation of potassium test solution was as described in the general method with the addition of a stabilizer in the pH solution. The organic reagent solution contained potassium ionophore 142 mM, and 7-decyl MEDPIN, 33.8 mM. The basic solution was NaOH, (50 mM). In the high pH reagent solution, the reagent concentrations were 1/3 of the original values. The working pH buffer solution was the liposome solution prepared above. The test solution contained THF 6.6%, ionophore, 9.47 mM, reporter, 2.25 mM, buffer, 80 mM, phosphatidyl choline, 2% w/v. It was tested with aqueous potassium solutions. The results are shown below and in Figure 11. .

| [K$^+$](mM) | OD |
|---|---|
| 0 | 1.181 |
| 0.19 | 1.218 |
| 0.38 | 1.257 |
| 0.57 | 1.293 |
| 0.76 | 1.337 |
| 1.14 | 1.400 |
| 1.33 | 1.436 |
| 1.52 | 1.475 |

Conclusions

1) The stabilizer can be any of a wide variety of surface active compounds; polymeric, (ethylene/maleic anhydride copolymer, poly(vinyl alcohol), polyvinylpyrrolidone, Triton X-100, polyoxyethylene-9-lauryl ether, water soluble cationic polyurethane, Pluronic® F68); negatively charged (ethylene/maleic anhydride, water soluble cationic polyurethane); zwitterionic (phosphatidyl choline) or neutral poly(vinyl alcohol), polyvinylpyrrolidone, Triton® X-100, polyoxyethylene-9-lauryl ether, or Pluronic® F68).

2) The preparation is completely resistant to centrifugation. Example B6 ($1 \times 10^6$ g-min), showing the reagents to be quantitatively present as a homogeneous solution.

## EXAMPLE C: VARIATIONS IN IONOPHORES

### Example C1: Calcium Test Solution

A homogeneous aqueous test solution for calcium was made using a podand known herein as Calcium Ligand II, (see abbreviation list for complete chemical name). The test solution was made following the procedure of Example A4. The organic reagent solution contained calcium ligand II 33.8 mM, 7-decyl MEDPIN, 25.4 mM, and ethylene/maleic anhydride copolymer, 7.5% w/v. This solution was added to 9 volumes of HEPES buffer (200 mM, pH 6.5) and further diluted three-fold with MES buffer (200 mM pH 6). The resulting test solution was red and a little cloudy. It contained THF, 3.3% v/v, ionophore, 1.13 mM, reporter, 0.85 mM, stabilizer, 0.25% w/v, buffer, 200 mM.

| $[Ca^{+2}]$ | OD |
|---|---|
| 0 | .611 |
| 0.2 | .748 |
| 0.4 | .851 |
| 0.6 | .956 |
| 0.8 | 1.045 |
| 1.2 | 1.184 |

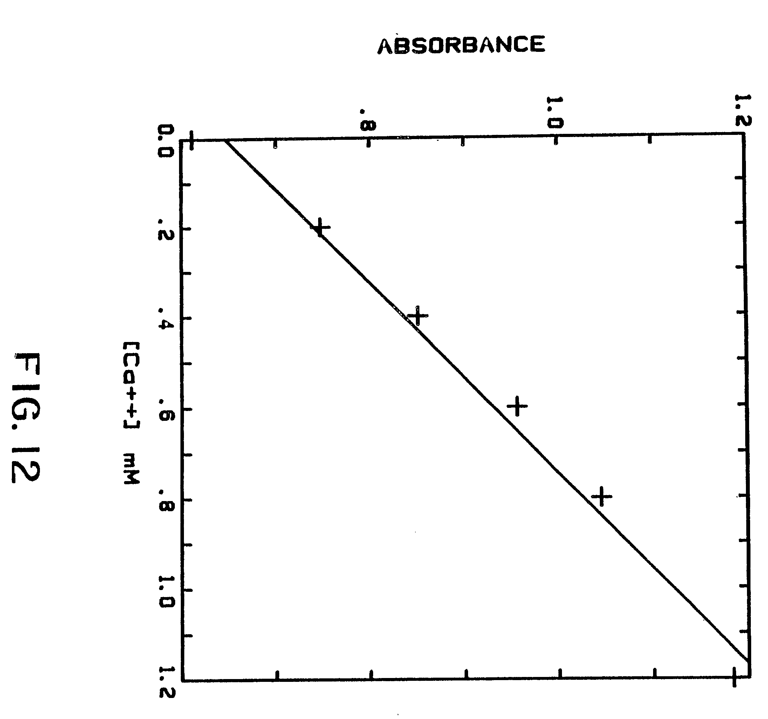

The data is shown in Fig. 12 and indicates a linear response to calcium ion can be obtained with test solutions prepared with the methods described herein.

### Example C2: Valinomycin as Ionophore

A test solution was prepared as in the general method with the addition of a stabilizer in the working pH solution. The organic reagent solution contained valinomycin, 17.5 mM, 7-decyl MEDPIN, 35 mM, and was red. The basic solution was NaOH, 50 mM. The high pH reagent solution produced was blue with a reagent concentration 1/10 of the original values. HEPES, 900 mM, pH 7.5, was used to prepare the working pH buffer solution which additionally contained 0.5% w/v of ethylene/maleic anhydride stabilizer. A clear, orange-brown test solution resulted containing THF, 3.3% v/v, ionophore, 0.58 mM, reporter, 1.17 mM, buffer, 600 mM, and stabilizer, 0.33% w/v.

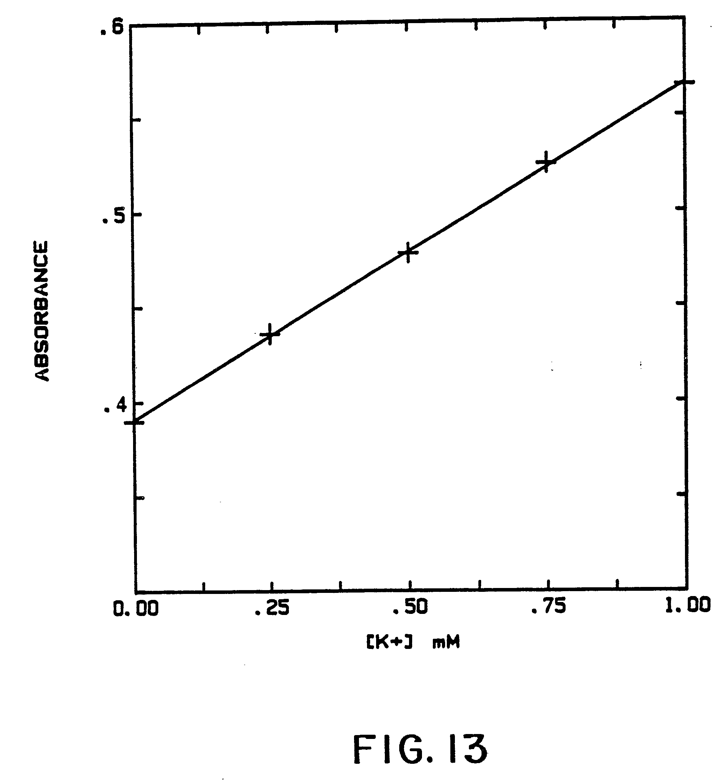

The dose response to aqueous potassium ion is shown below and in Fig. 13.

| $[K^+]$ (mM) | OD |
|---|---|
| 0 | .390 |
| 0.25 | .436 |
| 0.5 | .478 |
| 0.75 | .525 |
| 1.0 | .566 |

### Conclusion

It has already been shown that homogeneous aqueous test solutions can be prepared with two crown ethers which are generally known as cryptands. The sodium ionophore (Example A1) is a bis-crown ether and the potassium ionophore (Example A2) is a 15-crown-5 ether. Example C1 shows a podand can also be used and Example C2 shows an ionophorous antibiotic can be used.

## EXAMPLE D: VARIATIONS IN REPORTERS

### Example D1: Tetrabromophenolphthalein Decyl Ester (TBDE) as the Reporter

The test solution was prepared as described in the general method with the addition of a stabilizer to the basic solution. The composition of the yellow organic reagent solution was potassium ionophore, 71.4 mM and TBDE, 16.8 mM. The basic solution was NaOH, 200 mM, and contained ethylene/maleic anhydride copolymer 1% w/v, as the stabilizer. The high pH reagent solution was bright blue with reagent concentrations 1/10 of the initial values. Citrate, pH 4,1 M, was used to prepare the working buffer solution. A clear green test solution resulted which contained THF, 3.3% v/v, ionophore, 2.4 mM, reporter, 0.56 mM, stabilizer, 0.3% w/v, buffer, 670 mM.

Response to aqueous potassium ion samples was measured by absorbance at 620 nm, but in a 0.1 cm path length cuvette because of the high absorbance.

| $[K^+]$ (mM) | OD |
|---|---|
| 1 | 1.402 |
| 3 | 1.536 |
| 6 | 1.751 |
| 10 | 1.853 |

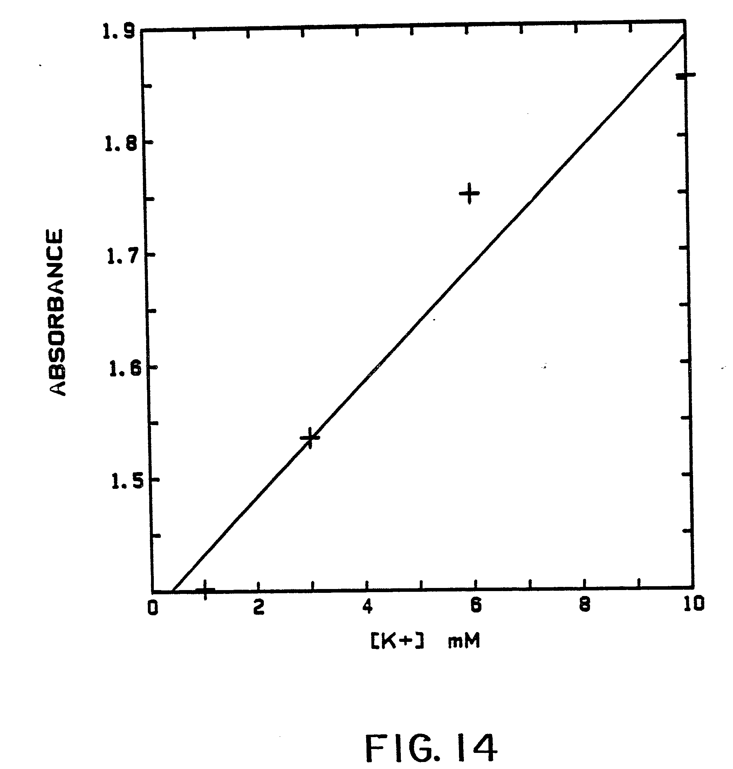

The data is shown in Fig. 14.

### Example D2: Methyl Eosin as the Reporter

A test solution was prepared as in Example D1. The composition of the organic reagent solution was potassium ionophore, 28.6 mM, and methyl eosin, 6.73 mM . The basic solution was NaOH, 200 mM, and contained ethylene/maleic anhydride copolymer 1% w/v, as the stabilizer. HEPES, pH 6.5, 1 M was used to prepare the working buffer solution. The composition of the test solution was THF 3.3% w/v, ionophore, 0.95 mM, reporter, 0.22 mM, stabilizer 0.3% w/v, and buffer, 670 mM.

Response to aqueous potassium ion was measured by absorbance at 560 nm, again in a 0.1 cm path length cuvette.

| $[K^+]$ (mM) | OD |
|---|---|
| 1 | .123 |
| 3 | .169 |
| 6 | .246 |
| 10 | .350 |

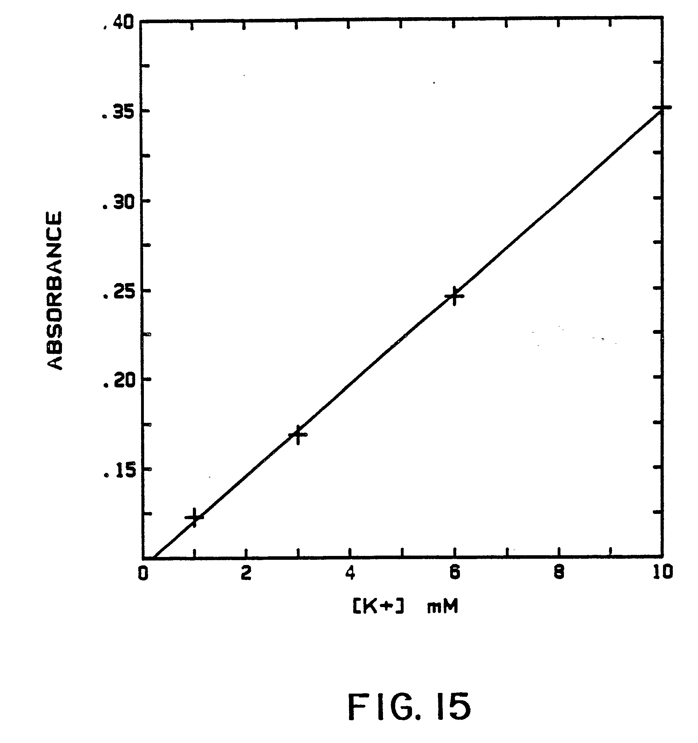

The data is shown in Fig. 15.

### Conclusion

The reporter can be a member of the indophenol (7-decyl MEDPIN), fluorescein (methyl eosin) or phthalein (TBDE) families of compounds.

Many modifications or variations of these examples can be made by one skilled in the art without departing from the spirit and scope of the invention which is solely defined by the claims. The invention effectively permits decentralized electro lyte testing to be accomplished in a standard colorimeter by

EP 0 319 863 A2

solving the problem of how to make a stable homogeneous reagent.

**Claims**

1. A method for producing a homogeneous aqueous test solution capable of measuring the concentration of an ion in an aqueous test sample, comprising:

a.) dissolving:

i.) an ionophore capable of forming a complex with an ion to be determined and

ii.) a neutral reporter having a dissociable proton which on dissociation produces a detectable response, in a basic solution having a pH which produces dissociation of the proton to form a high pH solution; and

b.) adjusting the pH of the high pH solution to substantially protonate the reporter and produce a test solution in a pH range wherein deprotonization occurs in response to the formation of an ionophore-ion complex.

2. The method of claim 1 wherein the dissolving step additionally includes a stabilizer.

3. A method for producing a homogeneous test solution capable of measuring the concentration of an ion in an aqueous test sample, comprising:

a.) dissolving

i.) an ionophore capable of forming a complex with an ion selected from the group consisting of Groups Ia and IIa of the Periodic Table of Elements, and

ii.) a neutral reporter having a dissociable proton which on dissociation produces a detectable response, in a water miscible organic solvent to produce an organic reagent solution;

b.) mixing the organic reagent solution with an aqueous solution having a pH which produces dissociation of the proton to produce a high pH solution; and

c.) adjusting the pH of the high pH solution to protonate the reporter and produce an aqueous test solution in a pH range wherein deprotonization occurs in response to formation of an ionophore-ion complex.

4. A method for producing a homogeneous test solution capable of measuring the concentration of an ion in an aqueous test sample, comprising:

a.) dissolving

i.) an ionophore capable of forming a complex with an ion to be determined;

ii.) a neutral reporter having a dissociable proton which on dissociation produces a detectable response upon formation of an ionophore-ion complex; and

iii.) a stabilizer,

in water miscible organic solvent to produce an organic reagent solution;

b.) adjusting the pH of the organic reagent solution with an aqueous solution having a pH in a pH range wherein deprotonization occurs in response to formation of the ionophore-ion complex, to produce an aqueous test solution containing less than 5% water miscible organic solvent.

5. A method for producing a homogenous test solution capable of measuring the concentration of an ion in an aqueous test sample, said method comprising:

a.) preparing a dried reagent by drying an organic reagent solution comprising,

i.) an ionophore capable of forming a complex with an ion;

ii.) a neutral reporter having a dissociable proton which on dissociation produces a detectable response upon formation of an ionophore-ion complex; and

iii.) a stabilizer; and

b.) dissolving the dried reagent in an aqueous solution having a pH in a pH range where deprotonization of the reporter occurs in response to formation of the ionophore-ion complex.

6. A method for preparing a homogeneous test solution capable of measuring the concentration of an ion in aqueous test sample, comprising:

a.) preparing a dried reagent by drying an organic reagent solution comprising:

i.) an ionophore capable of forming a complex with the ion; and

ii.) a neutral reporter having a dissociable proton which on dissociation produces a detectable response upon formation of an ionophore-ion complex;

20

b.) dissolving the dried reagent in an aqueous solution having a pH which produces dissociation of the proton to produce a high pH solution; and

c.) adjusting the pH of the high pH solution to protonate the reporter and produce an aqueous test solution in a pH range wherein deprotonization occurs in response to formation of the ionophore-ion complex.

7. A homogeneous aqueous test solution capable of measuring the concentration of an ion in an aqueous test sample, comprising:

a.) an ionophore capable of forming a complex with an ion;

b.) a substantially neutral reporter having a dissociable proton which on dissociation produces a detectable response; and

c.) a buffer, capable of providing a pH in the pH range wherein deprotonization occurs in response to the formation of an ionophore-ion complex.

8. A homogeneous aqueous test solution capable of measuring the concentration of an ion in a serum sample, comprising:

a.) an ionophore capable of forming a complex with an ion;

b.) a neutral reporter having a dissociable proton which on dissociation produces a detectable response;

c.) a buffer capable of providing a pH in the pH range wherein deprotonization of the reporter occurs in response to the formation of an ionophore-ion complex; and

d.) a stabilizer capable of maintaining the ionophore and neutral reporter in the aqueous solution without precipitation.

9. A lyophilized composition comprising:

a.) an ionophore capable of forming a complex with an ion, and

b.) a neutral reporter having a dissociable proton which on dissociation produces a detectable response.

10. A lyophilized composition comprising:

a.) an ionophore capable of forming a complex with an ion,

b.) a neutral reporter having a dissociable proton which on dissociation produces a detectable response, and

c.) a stabilizer.

FIG. I

FIG. 2

FIG. 3

FIG. 4

FIG. 5

FIG. 6

MS 1499

FIG. 7

FIG. 8

MS 1499

FIG. 9

FIG. 10

FIG. II

FIG. 12

FIG. 13

FIG. 14

MS 1499

FIG. 15

MS 14.99